(19) Europäisches Patentamt
European Patent Office
Office européen des brevets

(11) Publication number: **0 123 734 A1**

(12) **EUROPEAN PATENT APPLICATION**

(21) Application number: 83200616.7

(22) Date of filing: 29.04.83

(51) Int. Cl.³: C 07 J 41/00

(43) Date of publication of application: 07.11.84
Bulletin 84/45

(84) Designated Contracting States: NL

(71) Applicant: **Gist - Brocades N.V., Wateringseweg 1 P.O. Box 1, NL-2600 MA Delft (NL)**

(72) Inventor: **Van Leusen, Albert Mattheus, De Savornin Lohmanlaan 23, NL-9722 HC Groningen (NL)**
Inventor: **Van Leusen, Adriaan Marinus, Binnensingel 8, NL-9951 GD Winsum (Gr.) (NL)**

(74) Representative: **Huygens, Arthur Victor, Dr. et al, c/o Gist-Brocades N.V. Patent & Trademarks Department Wateringseweg 1 PO-box 1, NL-2600 MA Delft (NL)**

(54) **17-(Isocyano-sulfonylmethylene)-steroids, 17-(formamido-sulfonylmethylene)-steroids and their preparation.**

(57) The invention relates to 17-(isocyano-sulfonylmethylene)-steroids and a process for the preparation of these compounds. Furthermore the invention relates to 17-(formamido-sulfonylmethylene)-steroids, which compounds are, intermediates in the preparation of the 17-(isocyano-sulfonylmethylene)-steroids, and a process for the preparation of these compounds.

EP 0 123 734 A1

17-(Isocyano-sulfonylmethylene)-steroids, 17-(formamido-sulfonylmethylene)-steroids and their preparation.

The invention relates to 17-(isocyano-sulfonylmethylene)-steroids and a process for the preparation of these compounds. Furthermore the invention relates to 17-(formamido-sulfonylmethylene)-steroids, which compounds are, intermediates in the preparation of the 17-(isocyano-sulfonylmethylene)-steroids, and a process for the preparation of these compounds.

The invention concerns particularly 17-(isocyano-sulfonylmethylene)-steroids of the general formula

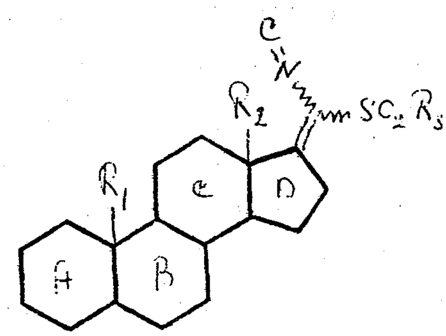

I

in which $R_1$ represents a hydrogen atom or a methyl group or is absent in the case of a double bond between $C_{10}$ and $C_1$, $C_5$ or $C_9$, $R_2$ represents a hydrogen atom or a methyl group, $R_3$ represents an alkyl or dialkylamino or an aryl group substituted or not substituted by one or more halogen atoms, alkyl, alkoxy, nitro or cyano groups, and the rings A, B, C and D optionally contain one or more double bonds, are substituted or not substituted by one or more hydroxy groups, amino groups, oxygen atoms, halogen atoms or alkyl, alkylene, alkoxy or alkoxyalkoxy groups and are disubstituted or not disubstituted by one or more epoxy groups, methylene groups, alkylenedioxy, alkylenedithio or alkyleneoxythio groups.

When $R_3$ represents an alkyl group, suitable alkyl groups are straight or branched alkyl groups having 1 to 8 carbon atoms, preferably 1-4 carbon atoms.

When $R_3$ represents a dialkylamino group, suitable dialkylamino groups are dialkylamino groups wherein the alkyl groups are the same or different and contain 1-8 carbon atoms, preferably 1-4 carbon atoms, or a dialkylamino group wherein the alkyl groups together with the nitrogen atom form a heterocyclic ring which optionally my contain an oxygen atom, the ring containing up to 8 ring atoms. Prefered dialkylamino groups are dimethylamino, diethylamino, pyrolidine and morfoline.

When $R_3$ represents an aryl group, a suitable group is phenyl substituted or not substituted by a halogen atom or an alkyl group, preferably a phenyl or p-methylphenyl group.

When the rings A, B, C and D contain one or more double bonds, these double bonds are preferably present between $C_1$ and $C_2$, $C_3$ and $C_4$, $C_4$ and $C_5$, $C_5$ and $C_6$, $C_6$ and $C_7$, $C_9$ and $C_{10}$, $C_9$ and $C_{11}$ and/or $C_{11}$ and $C_{12}$. More preferably the double bond is present between $C_9$ and $C_{11}$.

When two or more double bonds are present especially the following systems are prefered: $C_3$-$C_4$ and $C_5$-$C_6$, $C_4$-$C_5$ and $C_6$-$C_7$, $C_1$-$C_2$ and $C_4$-$C_5$, $C_1$-$C_2$, $C_3$-$C_4$ and $C_5$-$C_{10}$ and $C_1$-$C_2$, $C_4$-$C_5$ and $C_6$-$C_7$. Preferably there is also a double bond between $C_9$ and $C_{11}$.

When the rings A, B, C and D are substituted by a hydroxy group, suitable groups are 3-, 9-, 11-, 12- or 14-hydroxy groups.

When the rings A, B, C and D are substituted by an amino group, suitable amino groups are 3-alkylamino groups, preferably containing 1-4 carbon atoms, 3-dialkylamino groups wherein the alkyl groups are the same or different, each alkyl group preferably containing 1-4 carbon atoms, or amino groups in which the nitrogen atom together with the alkyl group form a heterocyclic ring, preferably containing 1-8 ring atoms, which ring optionally may contain an oxygen atom. Particularly preferred are dimethylamino, diethylamino, pyrolidine an morfoline.

When the rings A, B, C and D are substituted by an oxygen atom this oxygen atom is preferably present at $C_3$, $C_{11}$ or $C_{12}$.

When the rings A, B, C and D are substituted by a halogen atom, suitable halogen atoms are 6-, 9- or 11-fluorine, chlorine or bromine atoms, preferably 6- or 9-fluorine or chlorine atoms.

When the rings A, B, C and D are substituted by an alkyl group, suitable alkyl groups are 1-, 2-, 6-, 7- or 16 methyl groups, preferably 1-, 6- and 16-methyl.

When the rings A, B, C and D are substituted by an alkoxy group, suitable alkoxy groups are 3-, 9-, 11- or 12-alkoxy groups containing 1-4 carbon atoms, preferably 3-, 9-, or 11-methoxy or ethoxy groups.

When the rings A, B, C and D are substituted by an alkoxyalkoxy group, suitable groups are 3- or 11-methoxymethoxy, methoxyethoxy or tetrahydropyranyloxy.

When the rings A, B, C and D are disubstituted, suitable substituents are epoxy groups at $C_1$ and $C_2$ or $C_9$ and $C_{11}$ or a methylene group attached to $C_1$ and $C_2$ or a 3,3-alkylenedioxy a 3,3-alkylenedithio or a 3,3-alkyleneoxythio group. The alkylene group preferably contains 2 or 3 carbon atoms.

More particularly the invention relates to compounds in which $R_1$ and $R_2$ represents methyl or in which $R_1$ is absent, which are substituted by halogen, especially fluorine, or hydroxy at $C_9$ and a hydroxy or keto group at $C_{11}$, or containing functional groups, as a double bond or epoxy group between $C_9$ and $C_{11}$, which can be converted by methods known in the art into the group mentioned before, and which contain a keto group at $C_3$ and double bonds between $C_1$ and $C_2$ and/or $C_4$ and $C_5$, or containing functional groups which can be converted in the keto group and double bonds mentioned before.

The invention aims at the preparation of 20-sulfonyl-20-formamido-delta$^{17-20}$-steroids and 20-sulfonyl-20-isocyano-delta$^{17-20}$-steroids, because these compounds are valuable intermediates in the preparation of 21-hydroxy-20-keto-delta$^{16}$-steroids and 20-keto-delta$^{16}$-steroids as described in the simultaneously filed applications No. 0000000 and 0000000 entitled: "New process for the preparation of 21-hydroxy-20-keto-delta$^{16}$-steroids and new intermediate compounds formed in this process" and "New process for the preparation of 20-keto-delta$^{16}$-steroids and new intermediate compound formed in this process."

The invention also relates to a process for the preparation of the compounds as defined before by reacting a 17-oxo-steroid with a sulfonylmethylisocyanide and then dehydrating the resulting formamide to the corresponding isocyanide. Such a process is known. For instance European patent application No. 000 7672 discloses the said process applied to numerous ketones. Although not expected, it has been found that 17-(formamido-sulfonylmethylene-steroids and 17-(isocyano-sulfonylmethyl)-steroids could be prepared according to a corresponding process as described in European patent application 000 7672, starting with 17-oxo-steroids.

Therefore the invention also relates to a process for the preparation of 17-(isocyano-sulfonylmethylene)-steroid by reacting a ketone with a sulfonylmethylisocyanide, followed by dehydration of the resulting formamide, characterized in that the ketone is a 17-oxo-steroid.

In this connection the following is observed. The above mentioned European patent application contains one example (example 60) in which a steroid is used for the preparation of an alpha,bêta-unsaturated sulphonyl methyl formamide, and also the dehydration of this formamide to the corresponding isocyanide is described (example 26). In these examples the starting material was a 3-oxo-steroid.

However, as the 3-oxo-group of a steroid is more reactive than the 17-oxo-group, mainly due to steric reasons, it is not predictable for a man skilled in the art that these reactions also could be performed at the 17-oxo-group, especially not because of the known difficulties of reactions of p-methylphenylsulfonylmethyl-isocyanide in other type of reactions with sterically hindered ketones. It is surprisingly therefore, that alpha,bêta-unsaturated formamides and the corresponding isocyanides can be prepared from 17-oxo-steroids without problems.

It is further observed that reactions of p-methylphenylsulfonylmethylisocyanides with 17-oxo-steroids are known already, as appears from for

instance Tetrahedron 31, 2151 and 2157. In these publications the preparation of 17-alpha and 17-bêta cyano steroids is described. As a result of the already before mentioned steric hindrance of the 17-oxo-group, the reaction with p-methylphenylsulfonylmethylisocyanide to the 17-cyano steroids could only be performed by using fairly drastic reaction conditions. Although the mechanism for the formation of the cyano-compounds by reaction of p-methylphenylsulfonylmethyl-isocyanide with ketones is not known in details, it is generally believed that the above-mentioned alpha,bêta-unsaturated formamides, or more accurately their deprotonated anions, are intermediates in the formation of the cyano compounds. Isolation of the alpha,bêta-unsaturated formamides seemed to be not possible, because under the drastic reaction conditions, necessary for the first step in the reaction scheme in view of the steric hindrance of the 17-oxo-group, the formamides, once formed, would react immediately further into the before mentioned cyano-compounds. It was therefore surprising that it was still possible to isolate the desired alpha,bêta-unsaturated formamides instead of the cyanides which would be expected. This could be reached mainly by using sufficiently low temperatures, i.e. temperatures below -20°C, preferably at -40°C, preferably using tetrahydrofuran as solvent.

The invention also relates to the intermediate 17-(formamido-sulfonylmethylene)-steroids of the general formula:

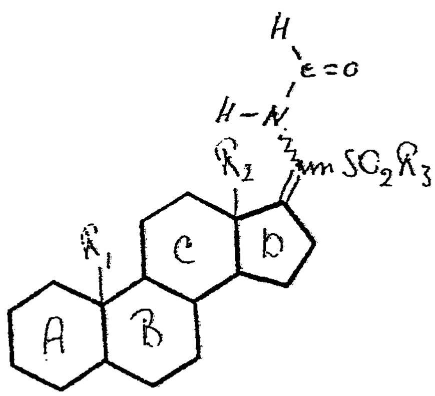

in which the substituents are as defined above.

Furthermore, the invention also relates to a process for the preparation of these formamides by reacting a ketone with a sulfonylmethyl isocyanide characterized in that the ketone is a 17-oxo-steroid.

As the 20-isocyano-20-sulfonyl-delta$^{16}$-steroids are intermediates in the preparation of the 20-keto-delta$^{16}$-steroids, the invention also relates to a process for the preparation of 20-keto-delta$^{16}$-steroids, characterized in that a 20-isocyano-20-sulfonyl-delta$^{16}$-steroid is dehydrated.

It is a further feature of the invention that both steps of the preparation process can be combined to a "one-pot-process".

If necessary in order to obtain the desired steroids or to improve the yield protective groups may be introduced. The protective group may be removed after the first or the second reaction step, the former is recommendable when the protective group affects unfavourably the second reaction step.

It is observed that the presence of a protective group can also be important when the isocyanides according to this invention are applied as intermediates for the preparation of 21-hydroxy-20-keto-delta$^{16}$-steroids or 20-keto-delta$^{16}$-steroids as described in the simultaneously filed applications. For example, methoxy and tetrahydropyranyloxy at the 3-position together with a double bond between $C_3$ and $C_4$ are protecting groups for the 3-oxo-group until their hydrolyses during the last reaction step in the preparation of the said hydroxy-keto-steroids.

Suitable 17-oxo-steroids for the process of the invention are those 17-oxo-steroids with the general formula:

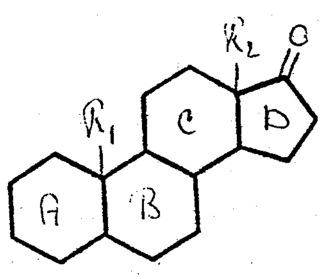

in which the steroid is as defined before.

For the reaction of the 17-oxo-steroids with the sulfonylmethylisocyanides the general reaction conditions can be used as described by Schöllkopf et al. Angew. Chemie, Int. Ed., 12, 407 (1973) and by Van Leusen et al, Recl. Trav. Chim. Pays Bas 98, 258 (1982).

Usually the reaction is carried out with a strong alkaline agent in an organic solvent, preferably in an inert gas atmosphere. Examples of useful strong alkaline agents are alkali metal alcoholates such as alkali metal t-butylates and alkali metal ethanolates, alkali metal hydrides, alkali metal amines, alkali metal alkyls and alkali metal aryls, in which the alkali metal is generally lithium sodium or potassium. Potassium t-butoxide is preferably used. The reaction is preferably carried out at lower temperature, e.g. between -20 and -80°C, preferably between -30 and -60°C, dependant on the solvent used too. The reaction is further preferably carried out in a polar organic solvent such as tetrahydrofuran, dimethylformamide, 1,2-dimethoxyethane, hexamethylfosfortriamide, dioxane, toluene or mixtures thereof. Tetrahydrofuran is preferred. The inert gas atmosphere is preferably a nitrogen or an argon atomsphere.

It will be appreciated that in principle the group $R_3$ of the sulfonylmethylisocyanides $R_3-SO_2-CH_2-N=C$ to be applied can be any group which does not interfere in the reaction. At least it will be possible to use those classes of sulfonylisocyanides which have been used already for this type of reactions. Examples of these classes are those compounds in which $R_3$ is aryl, alkyl or dialkylamino, whereby optionally one or more substituents can be present.

Suitable sulfonylmethylisocyanides are arylsulfonylmethylisocyanides in which the aryl group is optionally substituted by one or more halogen atoms, alkyl, alkoxy, nitro or cyano groups. Prefered arylsulfonylmethylisocyanides are phenylsulfonylmethylisocyanides in which the phenyl group is eventually substituted or not substituted by a halogen atom or a lower alkyl group. Particularly prefered are phenylsulfonylmethylisocyanide and p-methyl phenylsulfonylmethylisocyanide.

Any method for the preparation of isocyanides from formamides may be used, for example the reaction with fosforoxychloride. This reaction is preferably carried out at lower temperatures, e.g. between -50° and 25°C, preferably between -30° and -5°C. Other dehydrating agents, may however, also be used. Examples thereof are fosgene, thionyl chloride, cyanuryl chloride, alkyl and arylsulfonyl chlorides, a mixture of trifenylfosfine, carbon tetrachloride and triethyl amine, 2-chloro-3-ethylbenzoxazolium tetrafluoroborate or fosfor tri of pentachloride (see Ugi, Isonitril Chemistry, Acad. Press New York, 1971, pages 10 to 16) and difosgene (see Angew. Chemie, 89, 2671 (1977)). The dehydration is preferably carried out in the presence of an acid-binding agent, such as an amine. Examples of suitable amines are triethyl amine, substituted or unsubstituted pyridines, N-methylmorpholine, while other alkaline agents may be used too, such as potassium carbonate, sodium carbonate, potassium t-butoxide and in special cases even sodium carbonate, potassium t-butoxide. The dehydration is preferably carried out in an inert organic solvent, such as di-, tri- or tetra-chloromethane, ethyl acetate, dioxan, tetrahydrofuran, benzene, toluene, xylene, o-dichlorobenzene, acetone, 1,2-dimethoxyethane, bis(2-methoxyethyl)-ether, dimethylformamide or 1,2-dichloroethane or mixtures thereof.

The invention is illustrated by the following examples. In these examples TosMIC represents tosylmethyl isocyanide (p-methylphenylsulfonylmethyl isocyanide).

The specific rotation of the compounds was measured using light of the sodium D line.

Example 1a

Preparation of 3-methoxy-17-(isocyano-p-methylphenylsulfonyl-methylene) androsta-3,5-diene.

Potassium-t-butoxide (840 mg, 7.5 mmol) was added to dry tetrahydrofuran (50 ml) whereafter the suspension was cooled to -40°C. TosMIC (1.17 g, 6 mmol) was added to the suspension at -40°C. After 10 minutes stirring at this temperature 3-methoxyandrosta-3,5-dien-17-one (1.5 g, 5 mmol) was added. The mixture was stirred for two houres at -40/-30°C, followed by the addition of fosforic acid (615 mg, 7.5 mmol) at -35°C. After stirring for 10 minutes, triethylamine (7.5 ml, 54 mmol) and fosforoxytrichloride (1 ml, 11 mmol) were added at -35°C. The reaction mixture was stirred for one hour at 0°C, and poured into a mixture of 250 ml of ice water and 50 ml of brine. Extraction with $CH_2Cl_2$, drying over $MgSO_4$, evaporation in vacuum and crystallization from methanol afforded the alpha-bêta-unsaturated isocyanide (1.72 g, 3.6 mmol, 72%), m.p. 205°C (dec.); $(alpha)^{20}$ -85° (c 0.675, $CHCl_3$); IR (Nujol) 2140 (N=C), 1655, 1632, 1612 (C=C), 1600 (Ar), 1340 and 1162 ($SO_2$) $cm^{-1}$; $^1H$ NMR ($CDCl_3$) delta 0.8-3.2 (m), 0.96 (s, 3H), 2.42 (s), 3.5 (S, 3H), 5.0-5.3 (m, 2H), 7.15, 7.33, 7.64, 7.80 (ABq, 4H). Anal. calcd. for $C_{29}H_{35}NO_3S$ (477.67): C 72.92, H 7.39, N 2.93, S 6.71; found C 72.7, H 7.4, N 2.9, S 6.7.

Example 1b

Preparation of 3-methoxy-17-(formamido-p-methylphenylsulfonyl-methylen)androsta-3,5-diene.

Potassium-t-butoxide (1.26 g) was added to dry tetrahydrofuran (50 ml) whereafter the suspension was cooled to -50°C. TosMIC (1.17 g) was added to the suspension. After 10 minutes stirring at this temperature 3-methoxyandrosta-3,5-dien-17-one (1.5 g) was added. The mixture was stirred for 2.5 hours at -40/-55°C, followed by the addition of 0.92 g $H_3PO_3$. The reaction mixture was stirred for 20 minutes, and poured into a mixture of 250 ml of ice water and 50 ml of brine. Extraction with $CH_2Cl_2$ drying over $MgSO_4$, evaporation in vacuum and crystallization from hexane/$CH_2Cl_2$ afforded the alpha,bêta-unsaturated formadide (1.47 g, 59 %). IR ($CHCl_3$) 3396, 3367 (NH), 1699 (C=O), 1654, 1626, 1559 (C=C), 1316, 1141, ($SO_2$) $cm^{-1}$. NMR ($CDCl_3$) 0.945 (s, 6H), 2.41 (s, 3H), 3.53 (s, 3H), 5.16 (m, 2H), 7.2-8.2 (m, 6H).

Example 2

Preparation of 3-methoxy-17-(isocyano-p-methylphenylsulfonyl-methylene)estra-1,3,5(10)-triene.

The alpha-bêta-unsaturated isocyanide was prepared according to Example 1a starting from 3-methoxyestra-1,3,5(10)-triene-17-one (1.42 g, 5 mmol). The alpha-bêta-unsaturated isocyanide was precipitated from methanol as a gel. The methanol was removed and the gel was dried in vacuum. 1.62 g of the isocyanide was obtained (70%), m.p. 82-86°C (dec.). $(alpha)^{20}$ +46° (c 1.00, $CHCl_3$). IR (Nujol) 2150 (N=C), 1618, 1620 (Arom + C=C), 1390, 1342, 1162 ($SO_2$) $cm^{-1}$. $^1H$ NMR ($CDCl_3$) delta 1.1-3.2 (m), 2.42 (s, 3H), 3.70 (s, 3H), 6.53 (s, 1H), 6.68 (s, 1H), 7.00 (s, 1H), 7.21, 7.34, 7.69, 7.83 (ABq, 4H). Anal. calcd. for $C_{28}H_{31}NO_3S$ (461.62): C 72.85, H 6.77, N 3.03, S 6.95; found: C 73.1, H 7.2, N 2.85%.

Example 3

Preparation of 17-(isocyano-p-methylphenylsulfonylmethylene) androsta-1,4-diene-3-one.

The alpha-bêta-unsaturated isocyanide was prepared according to Example 1a, starting from androsta-1,4-diene-3,17-dione (1.42 g, 5 mmol). After crystallization from methanol at -20°C the isocyanide was obtained as a white solid, (1.35 g, 59%) m.p. 181-183°C (dec.). $(alpha)^{20}$ +181° (c 1.00, $CHCl_3$). IR (Nujol), 2140 (N=C), 1665 (C=O), 1630, 1610 (C=C), 1600 (Ar), 1380, 1335, 1160 ($SO_2$) $cm^{-1}$. $^1H$ NMR ($CDCl_3$): delta 0.8-3.2 (m), 1.0 (s), 1.21 (s), 2.43 (s), 6.00, 6.04, 6.20, 6.23 (2 x d 2H), 6.83, 7.00, (d, 2H), 7.18, 7.33, 7.65, 7.78, (ABq, 4H). Anal. calcd. for $C_{28}H_{31}NO_3S$ (461.62): C 72.85, H 6.77, N 3.03, S 6.95; found C 72.6, H 6.8, N 3.0, S 7.0%.

Example 4

Preparation of 3-methoxy-17-(isocyano-p-methylphenylsulfonyl-methylene)-androsta-3,5,9(11)triene.

The alpha-bêta-unsaturated isocyanide was prepared according to Example 1a, starting from 3-methoxyandrosta-3,5,9 (11)-trien-17-one (1.49 g, 5 mmol). The raw product was crystallized from 40 ml of methanol. Yield: 1.84 g (77%), m.p. 162-167°C. Two further crystallizations from methylenechloride/methanol (1:4) afforded a product with a melting point 172°C (dec.). $(alpha)^{20}$ -109° (c 1.00, $CHCl_3$). IR (Nujol) 2150 (N=C), 1660, 1640, 1615 (C=C), 1605 (Ar), 1380, 1345, 1270 ($SO_2$) $cm^{-1}$. $^1H$ NMR ($CDCl_3$): delta 0.8-3.3 (m), 0.90 (s), 1.09 (s), 2.41 (s), 3.50 (s, 3H), 5.0-5.55 (m, 3H), 7.20, 7.35, 7.65, 7.80, (ABq, 4H). Anal. calcd. for $C_{29}H_{33}NO_3S$ (475.65): C 73.23, H 6.99, N 2.94, S 6.74; found: C 72.7, H 7.0, N 3.0, S 6.7%.

Example 5

Preparation of 3-methoxy-11-bêta-hydroxy-17-(isocyano-p-methylphenylsulfonylmethylene)androsta-3,5-diene.

Potassium-t-butoxide (420 mg, about 3.75 mmol) was added to dry tetrahydrofuran under nitrogen. The suspension was cooled to - 40°C and then TosMIC (585 mg, 3 mmol) and 3-methoxy-11-bêta-hydroxyandrosta-3,5-dien-17-one were added. After two hours stirring at -40/-35°C $H_3PO_3$ (308 mg, 3.75) was added, followed after 10 minutes by triethylamine (7.5 ml, 54 mmol) and $POCl_3$ (1 ml, 11mmol). The $POCl_3$ was added in such a way (during a period of about five minutes) that the temperature remained below - 30°C. After two hours stirring at -30/-35°C the reaction mixture was poured into a mixture of 150 ml of water and 50 ml of brine, followed by extraction with successively 60,30 and 30 ml of $CH_2Cl_2$. After drying, filtration through $Al_2O_3$ (act. II-III) and evaporation of the solvent an oil was obtained. Addition of 20 ml methanol yielded crystals after cooling at -20°C. Drying above NaOH at 0.2 mm Hg yielded 940 mg (76%) of the isocyano-compound, m.p. 180°C (dec.). After two further crystallisations from 10 ml of $CH_2Cl_2/CH_3OH$ (1:5) the resulting substance had a m.p. of 188°C (dec.). (alpha)$^{20}$ -81° (c 1.00, $CHCl_3$). IR (Nujol) 3650 (OH), 2150 (N=C), 1655, 1630, 1615, 1598 (C=C + Ar), 1340, 1165 $cm^{-1}$($SO_2$). $^1H$ NMR ($CDCl_3$) delta 0.8 - 3.8 (m), 1.18 (s), 2.42 (s), 3.5 (s), 4.25-4.55 (m, 1H), 5.03 (s, 2H), 7.19, 7.33, 7.66, 7.79 (ABq, 4H). Anal.calcd. for $C_{29}H_{35}NO_4S$ (493.667): C 70.56, H 7.15, N 2.84, S 6.49: found C 70.1, H 7.2, N 2.7, S 6.5%.

Example 6

Preparation of 3-methoxy-9-alpha-fluoro-11-beta-hydroxy-17-(isocyano-p-methylphenylsulfonylmethylene)androsta-3,5-diene.

3-Methoxy-9-alpha-fluoro-11-bêta-hydroxy-androsta-3,5-dien-17-one (835 mg, 2.5 mmol) was treated in the same way as described in Example 5. The crude isocyano compound was

crystallized from 15 ml of methanol and washed with two portions of 5 ml of cold methanol. After drying 810 mg (63.5%) of the pure substance were obtained; m.p. 180°C (dec.), (alpha)$^{20}$ -87°(c 1.00, $CHCl_3$). IR (Nujol) 3580 (OH), 2170 (N=C), 1662, 1640, 1620, 1605 (C=C + Ar), 1345, 1165 ($SO_2$) $cm^{-1}$. $^1H$ NMR ($CDCl_3$) delta 0.8-3.3 (m), 1.17 (s), 1.24 (s), 2.42 (s), 3.50 (s, 3H), 4.05-4.60 (m, 1H), 5.10 (s, br, 2H), 7.21, 7.32, 7.67, 7.78 (ABq, 4H).

Example 7

Preparation of 3-methoxy-17-(isocyano-p-methylphenylsulfonylmethylene)androsta-3,5-dien-11-one.

3-Methoxyandrosta-3,5-diene-11,17-dione (785 mg, 2.5 mmol) was treated with the same chemicals as described in Example 1a, however using half the quantities mentioned there. After crystallization from 10 ml of methanol 875 mg (71%) of the isocyano compound are obtained; m.p. 195-205°C (dec.). Further purification by two crystallizations from $CH_2Cl_2$/methanol yielded a substance having a m.p. of about 220°(dec.) and an (alpha)$^{20}$ of -86.5°(c 1.00, $CHCl_3$). IR (Nujol) 2150 (N=C), 1705 (C=O), 1655, 1635, 1615 (C=C), 1595 (Ar), 1340, 1170 ($SO_2$) $cm^{-1}$. $^1H$ NMR ($CDCl_3$) delta 0.7-3.8 (m), 0.92 (s), 1.12 (s), 2.45 (s), 3.50 (s), 4.85-5.30 (m, 2H), 7.19, 7.33, 7.62, 7.77 (ABq,4H) Anal. calcd. for $C_{29}H_{33}NO_4S$ (491.65): C 70.85, H 6.77, N 2.85, S 6.52; found C 70.9, H 6.8, N 2.7, S 6.6%.

Example 8a

Preparation of 1-alpha,2-alpha-methylene-6-chloro-17-(formamido-p-methylphenylsulfonylmethylene)androsta-4,6-dien-3-one.

Potassium-t-butoxide (412 mg, 3.68 mmol)was added to dry THF (30 ml). The suspension was cooled to - 40°C under nitrogen. Then TosMIC (575 mg, 2.94 mmol) was added and after

its solution the temperature was lowered to -75°C, followed by the addition of 6-alpha-chloro-1-alpha,2-alpha-methylene-androsta-4,6-diene-3,17-dione (810 mg, 2.45 mmol). After 5 hours stirring TosMIC was no longer present and the formamide compound was isolated. The resulting substance had a m.p. of 259-260°C. $^1H$ NMR ($CDCl_3$) delta 0.6-0.9 (m, cyclopropyl), 1.002 (s, 3H), 1.204 (s, 3H), 2.46 (s, 3H) 6.3 (m, 2H) 7.3-8.4 (m, 6H).

Example 8b

Preparation of 1-alpha,2-alpha-methylene-6-chloro-17-(isocyano-p-methyl-phenylsulfonylmethylene)androsta-4,6-dien-3-one.

The formamide prepared according to example 8a (300 mg) was dissolved in 6 ml of THF and cooled to -20°C under nitrogen. Then triethylamine (0.8 ml) and $POCl_3$ ( 0.11 ml) were added, followed by stirring during half an hour at -20°C. The isocyanide was isolated and purified according to example 1a. The resulting pure substance had a m.p. of 144-151°C (browning at 118°C). IR ($CHCl_3$) 2110 (N=C), 1660 (C=O), 1615, 1601, (C=C), 1345, 1160 ($SO_2$) $cm^{-1}$. $^1H$ NMR ($CDCl_3$) delta 0.6-0.9 (m, cyclopropyl), 1.025 (s, 3H), 1.200 (s, 3H), 2.43 (s, 3H), 6.16 (m, 2H), 7.31-7.77 (m, 4H).

Example 9

Preparation of 3,3-ethylenedithio-17-(isocyano-p-methyl-phenylsulfonylmethylene)androsta-4-ene.

Potassium-t-butoxide (464 mg, 4.14 mmol) was added to dry THF 25 ml) and cooled to -60°C under nitrogen. Then TosMIC (0.659, 3.34 mmol) was added. After 10 minutes 3,3-ethylenedithio-androst-4-en-17-one (1 g, 2.76 mmol) dissolved in 5 ml THF was added followed by another 5 ml of THF. After two hours stirring at -60/-30°C acetic acid (0.24 ml,

4.2 mmol) was added at -40°C. After 10 minutes triethylamine (4.14 ml) and $POCl_3$ (0.55 ml) were added and the mixture was stirred during one hour (during the addition of the dehydrating agents the temperature raised to -10°C, stirring in a bath of 0°C). In order to complete the dehydration the same quantities of trietylamine and $POCl_3$ were again added and the mixture was again stirred for an hour. Then water was added and the aqueous layer was three times extracted with $CH_2Cl_2$. The collected $CH_2Cl_2$ solutions were dried over $MgSO_4$ and filtered. After evaporation of the solvent an oil was obtained. Crystallization from 20 ml of methanol afforded 0.85 g (yield 57%) of the isocyanide m.p. 213-216°C(dec.). IR ($CHCl_3$): 2107 (N=C), 1608, 1600 (C=C), 1337, 1155, ($SO_2$) $cm^{-1}$. $^1H$ NMR ($CDCl_3$) delta 1.03 (s, 3H), 1.10 (s, 3H), 2.45 (s, 3H), 3.30 (m, 4H), 5.50 (s, 1H) 7.2-7.95 (A Bq, 4H).

Example 10

Preparation of 3,3-ethylenedioxy-17-(ioscyano-p-methylphenylsulfonylmethylene)androsta-5-ene.

Potassium-t-butoxide (about 7.5 mmol) was added under nitrogen to THF (50 ml) and the mixture was cooled to -40°C. Then TosMIC (1.17g, 6 mmol) was added and after its solution 3,3-ethylenedioxyandrost-5-ene-17-one (1.65 g, 5 mmol). The reaction mixture was stirred at -30/-40°C for two hours. Though the TosMIC was completely used, the conversion of the steroid was not complete. Complete conversion was obtained by adding two times a further portion of 200 mg of TosMIC. Then $H_3PO_3$ (615 mg, 7.5 mmol) was added after about 20 minutes followed by addition of triethylamine (7.5 ml, 54 mmol) and $POCl_3$ (1 ml, 11 mmol). After stirring for one hour in a bath of 0°C and storing over night in a cool box the reaction mixture was

poured in 300 ml of a cold 10% solution of NaCl and extracted with $CH_2Cl_2$ (one time with 100 ml, then three times with 40 ml). The collected extracts were washed with a NaCl-solution (10%) and dried over $MgSO_4$. After evaporation a semi-solid residu remained, which yielded after purification with methanol and a trace of pyridine 2.07 g (89%) of the isocyanide, m.p. 183-186°C (dec.). IR ($CHCl_3$) 2105 (N=C), 1569, 1332, 1150 ($SO_2$) $cm^{-1}$. $^1H$ NMR ($CDCl_3$): delta 0.95 (s 3H), 1.03 (s, 3H), 2.47 (s, 3H), 3.93 (s, 4H), 5.36 (m, H), 7.40, 7.88 (ABq, 4H).

Example 11

Preparation of 3-bêta-(2'-tetrahydropyranyloxy)-17-(isocyano-p-methylphenylsulfonylmethylene)androst-5-ene.

3-Bêta-(2'-tetrahydropyranyloxy)-17-(formamido-p-methylphenylsulfonylmethylene)androsta-5-ene was prepared from 3-bêta-(2'-tetrahydropyranyloxyandrosta-5-en-17-one by reaction with TosMIC as described in Example 1a. $^1H$ NMR formamide ($CDCl_3$): delta. 0.887 (s, 3H), 0,977 (s, 3H), 2.41 (s, 3H), 3.3-4.1 (m, 2H), 4.68 (m 1H), 5.30 (m, 1H), 7.2-8.2 (m, 6H). The formamide compound (300 mg, 0.53 mmol) was dissolved in 6 ml THF and cooled to -20°C under dry nitrogen. Under stirring triethylamine (0.8 ml) and $POCl_3$ (0.11 ml) were added. After 30 minutes the reaction was completed. The reaction mixture was poured into an aqueous NaOH solution (50%, cooled in ice) and extracted with $CH_2Cl_2$ (one portion of 25 ml, 3 portions of 10 ml). The collected $CH_2Cl_2$ extracts were washed with a NaCl solution (10%) and dried on $MgSO_4$. After filtrating, evaporation of the solvent and drying under vacuum the isocyanide was obtained (283 mg); m.p. 146-152°C (browning at 137°C). IR ($CHCl_3$) 2106 (N=C), 1336, 1153 ($SO_2$), 1050 (-COC-)$cm^{-1}$. $^1H$ NMR ($CDCl_3$), 0.947 (s, 3H), 1.007 (s, 3H), 2.45 (s, 3H), 3.2-4.1 (m 2H), 4.67 (m 1H), 5.30 (m 1H), 7.37-7.82 (ABq 4H).

Example 12

Preparation of 1-alpha-methyl-3-methoxy-17-(isocyano-p-methylphenylsulfonylmethylene)-androsta-3,5-diene.

The alpha,bêta-unsaturated isocyanide was prepared according to Example 10, starting form 1-alpha-methyl-3-methoxyandrosta-3,5-dien-17-one (1.57 g, 5 mmol). After crystallization from methanol at -20°C the iscocyanide was obtained (1.33 g, 54%) m.p. 157-171°C. IR ($CHCl_3$) 2108 (N=C), 1338, 1156 ($SO_2$) $cm^{-1}$. NMR($CDCl_3$) delta 0.75 (d, 3H), 0.970 (s, 3H), 1.013 (s, 3H), 2.46 (s, 3H), 3.55 (s, 3H), 5.10 (m, 1H), 5.34 (m, 1H), 7.42-7.90 (ABq, 4H).

Example 13

Preparation of 3-methoxy-11-alpha-hydroxy-17-(isocyanoo-p-methyl-phenylsulfonylmethylene)androsta-3,5-diene.

Potassium-t-butoxide (160 mg, 1.5 mmol) was suspended in THF (12 ml) and cooled to -60°C. TosMIC (234 mg, 1.2 mmol) was added, followed after 10 minutes by 3-methoxy-11-alpha-hydroxyandrosta-3,5-dien-17-one (316 mg, 1.2 mmol). The clear solution was stirred for two hours at -50°C, followed by addition of triethylamine (3 ml) and $POCl_3$ (0.4 ml). The reaction mixture was stirred for 40 minutes at -40/-50°C and poured into a mixture of water and brine. After extraction with methylen chloride at pH 7, the organic layer was dried and evaporated. Crystallization from methanol afforded the title compound (260 mg, 52%), m.p. 235°C (dec.). $^1H$ NMR ($CDCl_3$) 0.98 (s, 3H), 1.10 (s, 3H), 1.49 (s, 1H), 2.47 (s, 3H), 3.55 (s, 3H), 4.07 (m, 1H), 5.10 (s, 1H), 5.22 (m, 1H), 7.36-7.82 (m, 4H), IR ($CHCl_3$) 3596 (OH), 2100 (N=C), 1655, 1630, 1610, 1594 (C=C), 1336, 1155 ($SO_2$) $cm^{-1}$.

Example 14

Preparation of 3-(N-morfoline)-17-(isocyano-p-methylphenylsulfonylmethylene)androsta-3,5-diene.

The title compound was prepared in the same way as described in Example 13, starting form 3-(N-morfoline)-androsta-3,5-dien-17-one (1.78 g, 5 mmol). Yield: 52%, m.p. 154-156°C. $^1H$ NMR ($CDCl_3$) 0.97 (s, 2 x 3H), 2.45 (s, 3H), 2.85-3.10 (m, 4H), 3.6-3.8 (m, 4H), 5.14 (d, 2H), 7.25, 7.41, 7.73, 7.88 (ABq, 4H). IR ($CHCl_3$) 2105 (N=C), 1600 (C=C)$cm^{-1}$, 1337, 1150 ($SO_2$) $cm^{-1}$.

Example 15

Preparation of 3-methoxy-17-(isocyano-p-methylphenylsulfonylmethylene)-19-nor-androsta-3,5-diene.

The title compound was prepared in the same way as described in Example 13, starting from 3-methoxy-19-nor-androsta-3,5-dien-17-one (725 mg, 2.5 mmol). Yield: 671 mg (55%), m.p. 163-168°C. $^1H$ NMR ($CDCl_3$ + DMSO) 1.0-3.2 (m), 0.97 (s, 3H), 2.47 (s, 3H), 3.55 (s, 3H), 5.22 (m, 2H), 7.30, 7.44. 7.74, 7.88, (ABq, 4H). IR ($CHCl_3$) 2105 (N=C), 1334, 1150 ($SO_2$)$cm^{-1}$.

Example 16

Preparation of 3-methoxy-6-chloro-17-(isocyano-p-methylphenylsulfonylmethylene)androsta-3,5-diene.

The title compound was prepared in the same way as described in Example 13, starting form 3-methoxy-6-chloro-androsta-3,5-dien-17-one (1.65 g). Yield: 1.6 g (56%), m.p. 180-181°C. $^1H$ NMR ($CDCl_3$) 0.997 (s, 6H), 2.46 (s, 3H), 3.61 (s, 3H), 5.60 (s, 1H), 7.34-7.82 (ABq, 4H). IR ($CHCl_3$) 2106 (N=C), 1645, 1618, 1598 (C=C).

Example 17

Preparation of 3-bêta-methoxymethoxy-17-(isocyano-p-methylphenylsulfonylmethylene)androsta-5-ene.

The title compound was prepared in the same way as described in Example 13, starting from 3-bêta-methoxymethoxy-androsta-5-en-17-one (1.68 g, 5 mmol). Yield: 0.78, m.p. 89-90°C. $^1H$ NMR ($CDCl_3$) 0.95 (s, 3H), 1.01 (s, 3H), 2.45 (s, 3H), 3.34 (s, 3H + 1H), 4.65 (s, 2H), 5.30 (s, 1H), 7.25, 7.40, 7.72, 7.86 (ABq, 4H). IR ($CHCl_3$) 2106 (N=C), 1335, 1147, ($SO_2$), 1597 (C=C), 1035 $cm^{-1}$.

Claims

1. Compounds of the general formula:

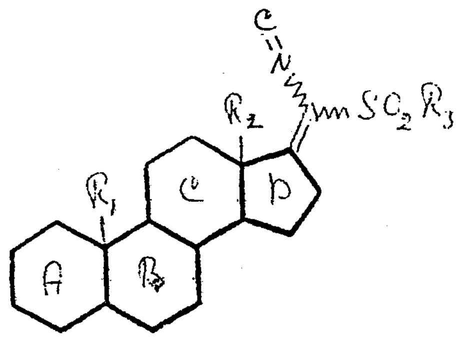

in which $R_1$ represents a hydrogen atom or a methyl group or is absent in the case of a double bond between $C_{10}$ and $C_1$, $C_5$ or $C_9$, $R_2$ represents a hydrogen atom or a methyl group, $R_3$ represents an alkyl or dialkylamino group, or an aryl group substituted or not substituted by one or more halogen atoms, alkyl, alkoxy, nitro or cyano groups, and the ring A, B, C and D optionally contain one or more double bonds, are substituted or not substituted by one or more hydroxy groups, amino groups, oxygen atoms, halogen atoms or alkyl, alkylene, alkoxy or alkoxyalkoxy groups and are disubstituted or not disubstituted by one or more epoxy groups, methylene groups, alkylenedioxy, alkylenedithio or alkyleneoxythio groups.

2. Compounds of the general formula:

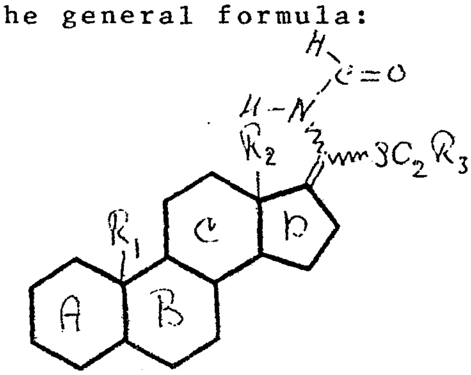

in which $R_1$ represents a hydrogen atom or a methyl group or is absent in the case of a double bond between $C_{10}$ and $C_1$, $C_5$ or $C_9$, $R_2$ represents a hydrogen atom or a methyl group, $R_3$ represents an alkyl or dialkylamino group, or an aryl group substituted or not substituted by one or more halogen atoms, alkyl, alkoxy, nitro or cyano groups, and the ring A, B, C and D optionally contain one or more double bonds, are substituted or not substituted by one or more hydroxy groups, amino groups, oxygen atoms, halogen atoms or alkyl, alkylene, alkoxy or alkoxyalkoxy groups and are disubstituted or not disubstituted by one or more epoxy groups, methylene groups, alkylenedioxy, alkylenedithio or alkyleneoxythio groups.

3. Process for the preparation of the compounds described in claim 1 by reacting a ketone with a sulfonylmethylisocyanide, followed by dehydration of the resulting formamide, characterized in that the ketone is a 17-oxo-steroid.

4. Process for the preparation of the compounds described in claim 2 by reacting a ketone with a sulfonylmethylisocyanide, characterized in that the ketone is a 17-oxo-steroid.

5. Process for the preparation of the compounds described in claim 1, characterized in that of the compounds described in claim 2 are dehydrated.

European Patent Office

# EUROPEAN SEARCH REPORT

0123734

Application number

EP 83 20 0616

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,A | EP-A-0 007 672 (GIST-BROCADES N.V.)<br>* Claim 1; examples 28,60 *<br>--- | 1,2 | C 07 J 41/00 |
| A | EP-A-0 023 856 (ROUSSEL UCLAF)<br>* Claims *<br>--- | 1,2 | |
| A | GB-A-2 079 756 (ROUSSEL UCLAF)<br>* Claims *<br>----- | 1,2 | |
| | | | TECHNICAL FIELDS SEARCHED (Int. Cl. 3) |
| | | | C 07 J 41/00 |

The present search report has been drawn up for all claims

| Place of search | Date of completion of the search | Examiner |
|---|---|---|
| THE HAGUE | 15-12-1983 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82